**Europäisches Patentamt**

**European Patent Office**

**Office européen des brevets**

(19)

(11) Publication number: **0 034 869**
**A2**

# (12) EUROPEAN PATENT APPLICATION

(21) Application number: **81200205.3**

(22) Date of filing: **20.02.81**

(51) Int. Cl.³: **A 61 L 2/00**
**A 61 L 2/20**

(30) Priority: **25.02.80 IT 2015680**

(43) Date of publication of application:
**02.09.81 Bulletin 81/35**

(84) Designated Contracting States:
**AT BE CH DE FR GB LI LU NL SE**

(71) Applicant: **Industrie Ottiche Riunite S.p.A.**
**Via Fratelli Bandiera 9**
**I-30175 Marghera-Venezia(IT)**

(72) Inventor: **Leopardi, Stefania**
**Viale Adriatico 2**
**I-00141 - Rome(IT)**

(74) Representative: **Faraggiana, Vittorio et al,**
**Ing. Barzanò & Zanardo S.P.A. Via Borgonuovo, 10**
**I-20121 Milano(IT)**

(54) **Method for sterilizing materials.**

(57) This invention relates to a method adapted to sterilize materials characterized in that said material is enclosed in the interior of a chamber having rigid and indeformable walls and in that the following sequence of steps is repeated one or more:

a) introducing into said chamber one or more gases, selected from among those which are capable of modifying the metabolic processes of the bacterial cells which are present, such as, principally, carbon dioxide, oxygen and nitrogen, until attaining a preselected pressure value in the interior of said chamber; b) producing a vacuum in the interior of said chamber until attaining a vacuum value which is variable within a range the minimum value of which is molecular vacuum; c) filling said chamber with one or more gases, selected from among those listed under a) above until attaining a preselected value of total pressure in the interior of said chamber, each of such stages being of such a duration as the specific nature of the bacteria concerned demands. The invention has also for its object to provide an apparatus which is adapted to carry out the above outlined method.

./...

Croydon Printing Company Ltd.

Fig. 1

"METHOD FOR STERILIZING MATERIALS"

As is well known, a material can be considered as being biologically sterile when it does not exhibit any vital bacterial form. The conventional sterilization methods consist in the application of such chemical and physical processes as to cause modifications in the bacterial cells in order that they may be inactivated.

In this connection, there can be mentioned, among the conventionally known sterilization procedures, those which exploit heat, which are based on the fact that an increase of the temperature above a certain value which is defined as the maximum optimum growth value for the bacterial cells has a lethal action on such cells, thus, dry heat sterilization procedures are known, which are generally carried out in ovens, for example for the treatment of Petri dishes, test tubes and like laboratory appliances, and also moist heat sterilization runs which are generally applied to all those materials which would not tolerate the high temperatures involved in dry heat sterilization treatments, or of those which use steam under pressure in an autoclave (for example for sterilizing those culturing media which are not altered when subjected to temperatures of about 15°C-120°C, i.e. those attained in an autoclave), or of the kind which uses live steam (for materials which do not tolerate temperatures above 100°C), or also those using steam baths (for example in the sterilization procedure known as pasteurization).

A serious problem which is common to all the sterilization processes which use heat, however, is the fact that there are different kinds of bacteria, more particularly the spore-forming species, which withstand heat even at high temperatures, or which, anyhow, acquire a character of resistance to heat under certain conditions, such as the presence of fats. Thus, if heat, on the one hand, is not an efficient sterilizing means towards many bacterial species, on the other hand, as outlined above, it is not indiscriminately applicable to any materials because it may alter the substances which are subjected to the action of high temperatures, irrespective of the fact that they are cell cultures, or laboratory appliances of materials sensitive to heat, such as plastics materials or rubber.

Another sterilization process which is widely used, especially in the cases in which heat is not applicable, but which, at any rate, can be adopted for liquids only, is the filtration, which consists in causing such liquids to flow through a porous body having pores of such a diameter as to bar the passage of bacteria therethrough, the bacteria being thus retained onto the filtering surface. In this connection, porous diaphragms of china, diatemaceous earth, or asbestos or filtering membranes of cellulose and cellulose esters are widely known. It is apparent that the possibility of applying such a process to liquids only, which in addition, must be clear and must be free flowing, is a considerable bar

3.

to the possibility of applying such a process.

Especially in the case in which materials which would be deteriorated by the use of high temperatures are to be sterilized, it is also known to employ ethylene oxide, either alone or in admixture with carbon dioxide in an autoclave. Problems inherent in the use of such a process, however, are the hazards of the use of that gas, which is highly inflammable, and the necessity of extending the treatment in time to provide an adequate efficiency thereof.

Other conventional sterilization techniques acts in the sense of modifying other physical and chemical parameters of the bacterium-environment system, such as the hydrogen-ion concentration; it is well known, in fact, the use of several acids as bactericides, but the behaviour of the several bacterial species towards changes of the pH of the environment in which they live is so varied (there are bacteria capable of growing at pH values below 1) that such sterilization process can be applied in a few specific cases only. A similar limitation applies also to the use, for bactericidal or bacteriostatic purposes, of surfactans, which are mainly active in the sense of modifying the value of the surface tension of liquids in which the bacterial cells are present in the sense of disturbing the vital functions of the cells concerned, and also the use of agents capable of inducing variations in the osmotic pressure; this, in general, inhibits the bacterial growth when high. Another known bactericidal process is the one which uses radiations, among which principally ultra violet, X-ray, alpha, beta and gamma radiations. Also these processes

4.

have considerable limitations to their use, such as, in the main, the necessity of costly apparatus and protection systems and the specific fields of their use.

Among the several chemicals having a bactericidal action, there can be mentioned, in the first place, the antibiotics, which are active at very low concentrations, but have very specific application fields and, above all, they are inefficient towards many bacterial strains which are resistant to the active chemical agent.

The main object of the present invention is thus to provide a sterilization method which can indiscriminately be applied to the most varied possible field of materials adapted to be treated to that purpose, be they cell cultures or laboratory or hospital appliances, said method being highly efficient towards the highest possible number of bacterial species, the problems of selectivity and specificity of use of the conventional sterilization processes being thus set aside.

An object of this invention is also to define a method of sterilization having the features outlined above, which comprises operations which are simple to be performed, principally in the sense of dispensing with the use of intricate and costly apparatus, and which can be directly applied to the most varied fields.

It has now been found, and this is the subject matter of the present invention, that the objects outlined above can be very advantageously achieved by a method adapted to the sterilization of various materials, said method being characterized in that said material is enclosed in the interior of a chamber having rigid and indeformable walls and the following sequence of

5.

steps is repeated once or more: a) introducing into said chamber one or more gases, selected from among those capable of modifying the metabolic processes of the bacterial cells which are present, such gases being principally carbon dioxide, oxygen and nitrogen until attaining a preselected value of the pressure in the interior of said chamber; b) producing a vacuum in the interior of said chamber until attaining a value of vacuum variable within a range the lower limit of which is molecular vacuum, and c) filling said chamber with one or more gases, selected from among those defined for step a) above until attaining a preselected pressure value in the interior of said chamber, each of said steps lasting for a period of time which is preselected as a function of the specific nature of the bacterial species concerned.

It is pointed out herein that the several parameters concerned in the different steps of the method hereof, such as pressure, temperature and time, can be varied within a wide range; so it is, obviously, because it is possible to establish at will one or more of such parameters, the remaining ones being bound thereto as a function of the various thermodynamic laws of the reversible and irreversible processes under the equilibrium conditions.

This invention is also concerned with an apparatus which permits to perform the method outlined above, so that the apparatus is substantially comprised of a chamber having rigid and indeformable walls, which is adapted to enclose in a sealtight manner a material to be subjected to sterilization, means for feeding one or more gases to said chamber, and means for producing a vacuum in the interior of said chamber.

An apparatus which lends itself particularly well to carry out the process of this invention is the one which is depicted in the accompanying drawings.

It is pointed out, however, that the process according to this invention can be equally well performed by any similar apparatus which is adapted to the purpose.

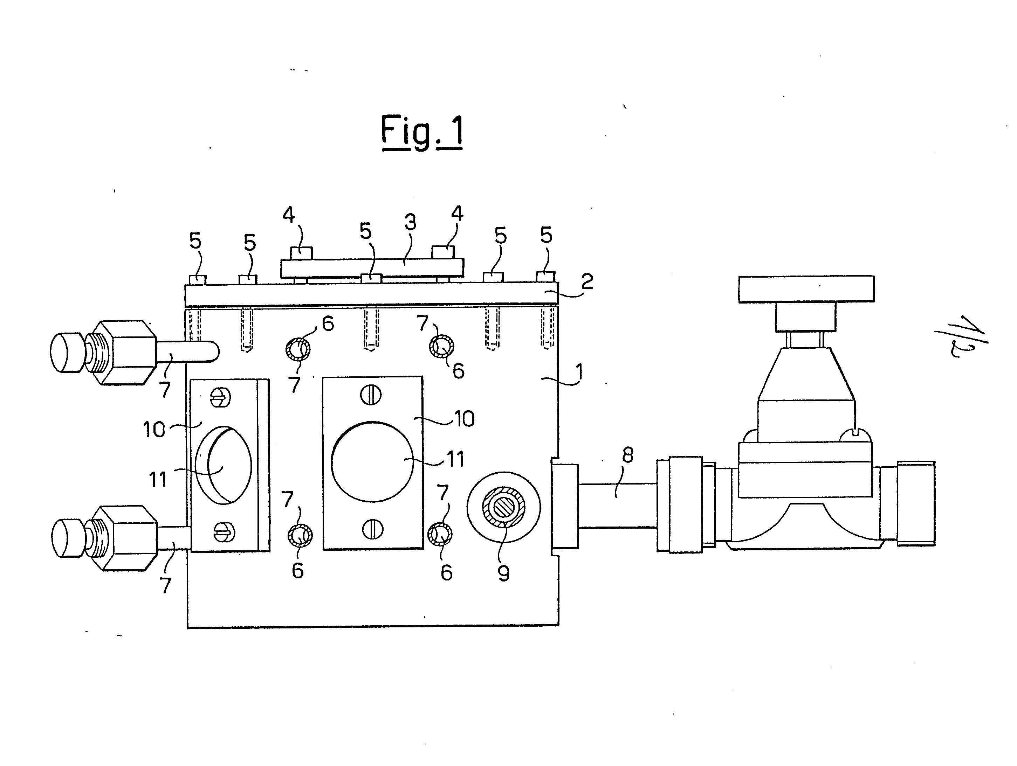

Figure 1 is a side elevational view, taken from the line I-I of fig. 2, of an apparatus capable of performing the process of this invention.

Figure 2 is a top plan view of the same machine.

The apparatus shown in the drawings is comprised of a cylindrical chamber 1, made of a material which is adapted to withstand high pressure and also molecular vacuum conditions and can be, for example, of steel or a very hard plastics material such as a polymethylacrylate.

Said chamber has, at its top, a lid for introducing and withdrawing the samples to be sterilized, the lid being comprised of two complementary pieces 2 and 3 which are adapted to provide a tight seal when the chamber is sealed, that is when the apparatus is in readiness for operation. The sealing of the chamber by said lid takes place by screwing, by the agency of the screws 4 provided for the piece 3 and the screws 5 provided for the piece 2.

The chamber 1 has inlets 6, having valve couplings 7 for feeding a gas stream thereinto or also to have access to the chamber interior for carrying out additions or withdrawals relative to the samples to be sterilized.

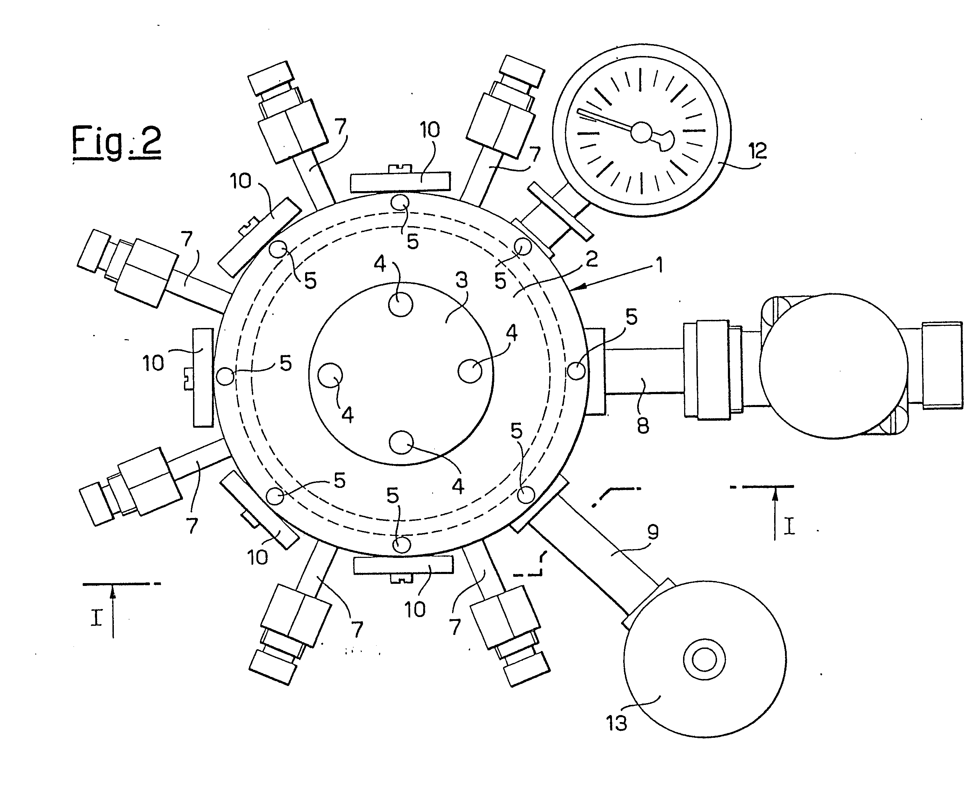

Chamber 1 has also, associated therewith, a conduit 8 to be connected to a vacuum pump, and also a shaft 9 connected to a motor 13 which is shown diagrammatically in figure 2.

The shaft 9 enters the chamber interior and is intended to effect, according to known constructional and functional details, a movement of rotation or stirring of the material to be sterilized during the several stages of the process.

To the chamber walls are secured a number of frames or covers 10 having windows 11 to check at every instant of time the operative conditions of the process such as viscosity, density, concentration, pH of the sample. To this purpose, the covers may have associated therewith thermocouples for controlling and measuring such parameters by means of appropriate instruments. At 12, finally a pressure gauge is shown to measure the pressure in the interior of the chamber 1.

The operation of the apparatus shown in the drawings is extremely simple and consequential: once the sample has been introduced into the chamber and that the latter has been sealed by its lid, the gas is fed through appropriate intake tubes coupled to the fittings 7 connected to the openings 6. The vacuum in the chamber is produced via the duct 8 which has a cock for connection to and disconnection from the vacuum pump.

During the several stages of the method, as outlined above, the sample in the interior of the chamber can be stirred or rotated, preferably with a slow motion: in the case of cellular cultures, such a motion of the

8.

sample has the function of preventing any undesirable settling or clumping.

In order that the objects and the features of the operation of the method according to the invention may be better understood, a few examples of practical application will be reported hereinafter, which, however, should not be construed as limitations of the field of application of the method or of the ranges of values of the several working parameters indicated therein.

EXAMPLE 1

It is desired to sterilize a culture of adult or foetal blood which has been prepared according to the conventional procedure but which does not contain any antibiotics.

A sample of such a standard culture is introduced in the chamber of the apparatus described hereinbefore: the interior of the tightly sealed chamber is then fed with a continuously flowing stream of 400 mls of $CO_2$ until attaining a pressure of 5 atm at the temperature of 37.5°C.

The pressure in the interior of the chamber is then reduced to a value of about 5 mmHg and this vacuum is maintained for about 30 minutes.

The interior of the chamber is then fed with a gas mixture composed by $CO_2$, $O_2$ and $N_2$, the respective percentages by weight being 75%, 15% and 10% until attaining a total pressure of 5 atm (still at 37.5°C) in the interior of the chamber, these conditions being maintained for 30 minutes.

Such a treatment is performed on the blood culture at the start (zero time) and from the thirteenth to the fifteenth hour.

EXAMPLE 2

It is desired to sterilize Petri dishes. The latter, after having been washed and dried, are placed in the chamber of an apparatus according to the invention, having steel walls.

In the tightly sealed chamber a mixture of $CO_2$ and $N_2$ is fed in a volume ratio of 4 to 1, until the air initially contained in the chamber has been purged. The gas mixture is allowed to stand in the interior of the chamber during 30 minutes approx. Molecular vacuum is then produced in the chamber and maintained for 30 additional minutes, approx.

The chamber is now fed with the same gas mixture of nitrogen and carbon dioxide as indicated above until reaching a pressure of 25 atm and the mixture is allowed to stand in the chamber for a period of about 30 minutes.

EXAMPLE 3

In the particular case to be described in this example, it is desired to disclose the mechanism and the intermediate stage of a process of conversion of a healthy cell of venous blood into a pathological cell with a view to detecting the factors which are responsible for such a conversion. The sterilization process according to the present invention has proved to be extremely useful to this purpose.

A cell culture of peripheral venous blood of a healthy person is prepared and introduced in the sterilization chamber according to the invention. Sterilization is carried out as described in Example 1 hereof. On completion of the sterilization process, the culture

10.

in the interior of the chamber is subjected to the following operative stages: a mixture of oxygen rich (90%) air is introduced until a pressure of 1.5 atm is attained, for about 60 minutes; vacuum is produced and maintained for about one hour; nitrogen is introduced under a pressure of 2.5 atm for a period of 15 minutes, starting from the fourth hour of incubation and to the tenth hour; the dishes containing the culture are rotated at a constant rate of 30 rpm; from the 13th to the 15th hour the rotational speed is halved. $CO_2$ is now introduced under a pressure of 5 atm for about 20 minutes. Vacuum is produced in the chamber interior again and $CO_2$ is introduced once more under a pressure of 5 atm.

The culture is then irradiated with laser light at an infrared wave length up to the 24th hour of processing. Once that the dishes have been removed and the slides are prepared, whereafter the culture is examined under the photomicroscope.

A procedure such as described above might be of great interest for the study of phenomena of resistance by bacteria to chemical antibiotics. It becomes thus possible to make conspicuous the transfer of intracellular material from a cell endowed with resistance properties to a sensitive cell, thus labelling the factor which is responsible for such a resistance character.

In order to give an evidence of the advantage of the sterilization process according to the invention, there is reported hereinafter a comparison test which has been performed on a line of cellular cultures of venous whole blood (peripheral) of grown-up individuals, which has been sterilized with the method according to Example 1 hereof.

A sample lot, to be called "A" hereinafter, of 10,000 cell cultures of the type referred to above, sterilized according to the invention, has been prepared in parallel with a like lot, to be called "B" hereinafter, of cultures which differ from the former only in that they had been treated, rather than with the sterilization process according to this invention, with an association of antibiotics (penicillin + streptomycin).

The leucocyte count in each culture of both lines A and B in the order of magnitude of $4.5 \cdot 10^3$. Lymphocytes have been estimated with the well known leucocyte formula: their amount at the zero instant of time was 23% relative to the population of the white cells. The mitotic index has been calculated according to the Sasaki and Norman method, that is, at the 60th hour of incubation.

The cultures in which no cell division, or mitosis, has been experienced have been counted, said lack of division having been caused by the presence of bacteria in the relevant culturing media.

In the cultures of the lot "A" no mitosis has been detected in 50 cultures out of 10,000, that is the 0.5%.

In the cultures of the lot "B" no mitosis has been detected in 200 cultures out of 10,000, that is the 2%. The result has thus been that the sterilization process according to this invention permits that a deactivation may be obtained for the bacterial activity, relatively to the cellular reproduction, which is considerably improved over that exerted by a sterilizing

12.

agent of conventional type, such as associations of antibiotics.

The mechanism of action of the sterilization process of this invention might be explained, in its essentials, as follows:

The introduction into the chamber of a gas mixture, selected consistently with the nature of the material to be sterilized in the first stage a) of the method described herein provides a gaseous atmosphere which, when maintained under preselected conditions of temperature and pressure for an equally preselected time, is capable of selectively acting upon the metabolic processes of the bacterial cells and thus of bringing about modifications of their structures.

In this connection, if example 1 hereof is duly considered, the introduction of $CO_2$ until attaining a pressure of 5 atm in the chamber in the stage a) of the method referred to above, has the result of causing an initial deactivation of the bacteria which are present, and, selectively, of the aerobic species, while stabilizing the blood culture so treated prior to passing to the subsequent sterilization stages.

During stage a) the bacterial cells are thus, as a rule, subjected to a gas atmosphere capable of acting thereupon in a selective biological way: in addition, the gases impart a pressure onto the cellular walls.

In the subsequent stage, b), vacuum is rapidly provided in the interior of the chamber: the sudden transition from pressural conditions to molecular vacuum conditions has the effect of subjecting the cellular walls to a considerable strain.

Microorganisms, as a rule, have proven to be incapable of withstanding a physical stress of that kind: it can be surmised that, on such an occasion, and especially in correspondence with certain active sites of the cell walls, a membrane breakdown occurs with attendant spurt of intracellular material, any bacterial activity being thus neutralized.

Summing up, it can be said that the production and the maintenance of a vacuum in the interior of the chamber has the effect of modifying the surface tension of the materials subjected to sterilization. It is difficult, however, to explain the exact mechanism whereby such a phenomenon displays a bacteriostatic or a bactericidal action: it should be taken into account, for example, that the variation of the surface tension at the contact surface between a liquid (i.e. the solution to be sterilized) and the gas is widely different from that obtaining between the cell wall and the surrounding liquid.

The last stage of the process suggested herein effects, concurrently, a "scrubbing" of the material and a final selective deactivation on the bacteria which are still present, with no air being virtually present of that initially contained in the chamber.

In the stage c) of example 1, while $CO_2$ effects a further deactivation of the bacteria, oxygen is active upon the obligate anaerobic species which live only at low concentration of that gas, and nitrogen blocks the metabolic activity of the anaerobic bacteria.

When a higher degree of sterilization is desired, the process of this invention provides for a repetition

14.

of the stages a), b) and c) for many times.

CLAIMS :

1.   A method adapted to the sterilization of a material characterized in that said material is enclosed in the interior of a sealtight chamber having rigid and indeformable walls and in that the following sequence of stages is repeated one or more times:

a) introducing into said chamber one or more gases, selected from among those which are capable of modifying the metabolic processes of the bacterial cells concerned, such as principally carbon dioxide, oxygen and nitrogen until attaining a preselected value of pressure in the interior of said chamber;

b) producing a vacuum in the interior of said chamber, variable within a range the lower value of which is molecular vacuum;

c) filling said chamber with one or more gases, selected from among those defined above under a) until attaining a preselected value of total pressure in the interior of said chamber,

each of said stages being caused to last for a period of time which is preselected as a function of the specific nature of the bacteria concerned.

2.   An apparatus adapted to perform the sterilization method as claimed in claim 1, characterized in that it comprises a chamber having rigid and indeformable walls and adapted to enclose in a sealtight manner a material to be subjected to said sterilization, means for feeding one or more gases to said chamber, means for producing a vacuum in the interior thereof.

3.   An apparatus according to claim 2, characterized in that it comprises means for impressing a rotation or

2.

a stirring motion to said material to be sterilized.

4. An apparatus according to claim 2, characterized in that it comprises means for having access to the interior of said chamber with specially provided instruments to carry out operations of sampling, or introduction of substances contained therein during progress of said sterilization process.

5. An apparatus according to claim 2, characterized in that it comprises means for controlling and measuring the operational parameters in the interior of said chamber.

6. A method and an apparatus substantially as hereinbefore described and illustrated in the accompanying drawings.

# Fig. 1

Fig.2

2/2

0034869